# EUROPÄISCHE PATENTSCHRIFT

(11) **EP 1 995 594 B1**
(45) Veröffentlichungstag und Bekanntmachung des Hinweises auf die Patenterteilung: **08.07.2009**
(21) Anmeldenummer: 07010017.7
(22) Anmeldetag: 19.05.2007
(51) Int. Cl.: G01N 33/487

(54) **Analysehandgerät zum Untersuchen einer Probe**
Handheld analysis device for investigating a sample
Appareil d'analyse portable pour tester des échantillons

(43) Veröffentlichungstag der Anmeldung: 26.11.2008
(73) Patentinhaber: Roche Diagnostics GmbH, 68305 Mannheim (DE); F. Hoffmann-La Roche AG, 4070 Basel (CH)
(72) Erfinder: Schulat, Jochen, 68305 Mannheim (DE); Albrecht, Gertrud, 38239 Mannheim (DE); Kern, Bernhard, 69123 Heidelberg (DE)
(74) Vertreter: Jany, Peter

(56) Entgegenhaltungen:
- EP-A- 0 779 983
- EP-A- 1 508 807
- WO-A-20/04057345
- WO-A-20/05065828
- WO-A-20/05085840

## Beschreibung

Die Erfindung betrifft ein Analysehandgerät zum Untersuchen einer Probe, insbesondere einer biologischen Flüssigkeit, hinsichtlich eines medizinisch bedeutsamen Bestandteils und ein Verfahren zum Betreiben eines solchen Analysehandgeräts. Das Analysehandgerät enthält eine Analyseeinrichtung, eine Anzeigeeinrichtung, ein Gehäuse, das eine Ladeöffnung zum Aufnehmen eines auswechselbaren Magazins, das analytische Verbrauchsmittel, insbesondere Teststreifen, enthalten kann. Das Magazin kann mehrere Kammern aufweisen und beispielsweise als Trommelmagazin ausgebildet sein, wobei die Kammern jeweils eine Öffnung an einer Stirnseite des Magazins aufweisen, die jeweils mit einer Siegelfolie verschlossen sein können. Ferner umfasst das Analysehandgerät eine Entnahmeeinrichtung zum Entnehmen eines der analytischen Verbrauchsmittel aus dem Magazin, mittels der eines der Verbrauchsmittel aus einer der Kammern des Magazins herausbefördert und auf einen Förderweg gefördert werden kann. Dabei oder anschließend wird das analytische Verbrauchsmittel auf dem Förderweg zum Analysesensor der Analyseeinheit gefördert, um dort eine Analyse durchzuführen. Eine Prüfeinheit stellt die korrekte Positionierung eines analytischen Verbrauchsmittels auf dem Förderweg fest.

Für die chemische und biochemische Analyse von festen und flüssigen Probenmaterialien haben sich in darauf spezialisierten Labors und insbesondere auch für den Einsatz außerhalb fester Labors trägergebundene Schnelltests etabliert. Solche trägergebundene Schnelltests basieren auf einer eigens entwickelten Trockenchemie und sind trotz der oftmals komplexen Reaktion unter Beteiligung empfindlicher Reagenzien selbst von Laien einfach und unkompliziert durchzuführen.

Ein bekanntes Beispiel für trägergebundene Schnelltests sind Testelemente für die Bestimmung des Blutglucosegehalts bei Diabetikern. Diagnostische Testelemente, die streifenförmig ausgebildet sind, werden auch als Teststreifen bezeichnet. Bekannte Ausführungsformen sind z.B. Einfeld- oder Mehrfelder-Teststreifen für die Urinanalytik und diverse Indikatorpapiere. Da neben Testelementen in Streifenform auch andere Formen trägergebundener Tests existieren, spricht man allgemeiner von analytischen Verbrauchsmitteln, wozu beispielsweise auch Lanzetten oder Probeentnahmeelemente zählen.

Derartige analytische Verbrauchsmittel werden in einem Analysehandgerät verwendet, das beispielsweise mit einer optischen Analyseeinrichtung eine Verfärbung eines Teststreifens photometrisch auswertet. Die analytischen Verbrauchsmittel sind in einem Trommelmagazin gelagert, wie es beispielsweise in der EP 1 022 565 A2 beschrieben ist. Ein solches Trommelmagazin weist mehrere ringförmig angeordnete Kammern auf, die analytische Verbrauchsmittel enthalten können. Die Kammern weisen jeweils eine Einschuböffnung und eine Entnahmeöffnung an gegenüberliegenden Stirnseiten des Trommelmagazins auf. Diese Öffnungen sind jeweils mit einer Siegelfolie verschlossen, um die analytischen Verbrauchsmittel vor schädlichen Umwelteinflüssen, wie z.B. Licht, Feuchtigkeit oder Staub, zu schützen.

Bei bekannten Analysehandgeräten, beispielsweise dem "Accu-Chek® Compact Blutzuckermessgerät" der Roche Diagnostics GmbH, Mannheim, 2001, wird durch Betätigung einer Entnahmeeinrichtung geprüft, ob sich in einer Kammer des Trommelmagazins noch ein analytisches Verbrauchsmittel befindet oder dieses bereits entnommen wurde. Das bekannte Analysehandgerät hat zwei Prüfstromkreise. Ein erster Prüfstromkreis wird von einem an einer Stößelstange der Entnahmeeinrichtung angebrachten Mitnehmer geschlossen, wenn diese Stößelstange in eine Kammer des Trommelmagazins eingeführt wird. Befindet sich in der Kammer ein Verbrauchsmittel, so wird es von der Stößelstange herausgeschoben und dabei von dem Verbrauchsmittel ein Schalter betätigt, der einen zweiten Prüfstromkreis schließt. Führt ein Betätigen der Entnahmeeinrichtung also nur zu einem Schließen des ersten Prüfstromkreises, nicht aber zum Schließen des zweiten Prüfstromkreises, so bedeutet dies, dass die betreffende Kammer des Trommelmagazins leer ist.

Aus dem europäischen Patent EP 0 779 983 B1 ist ein analytischer Nachweisstreifen mit Orientierungsindex und ein Analysegerät hierfür bekannt. Mit Hilfe des Orientierungsindexes, der mit Hilfe eines optischen Sensors erfasst wird, wird die Positionierung des Nachweisstreifens und damit die Anwesenheit des Nachweisstreifens erfasst und abhängig davon die Durchführung der Analyse gesteuert. Durch das Aufbringen des Orientierungsindexes auf dem Nachweisstreifen ergibt sich ein aufwändiger Aufbau des Nachweisstreifens, der zu Fehlfunktionen insbesondere aufgrund von Verschmutzungen führen kann.

Weiterhin ist aus der europäischen Patentanmeldung EP 1 508 807 A2 eine Positioniereinrichtung für ein Testelement bekannt, bei dem mittels einer elektrischen Schaltkomponente, die in Richtung des Testelementes verschiebbar ausgebildet ist und die mit einem dem Testelement zugewandten konisch zulaufenden Ende versehen ist, signale erzeugt werden. Dieses konisch zulaufende Ende korrespondiert mit einer Ausnehmung in dem Testelement, das mit dem Ende dahingehend zusammenwirkt, dass die Position des Testelements in dem Analysegerät so eingenommen wird, dass eine sichere Analyse des Testelementes erreicht werden kann. Die Positioniereinrichtung ist dabei so ausgebildet, dass durch die verschieblich ausgebildete Schalterkomponente in Abhängigkeit von der Anwesenheit bzw. der richtigen Positionierung des Testelementes elektrische Kontakte geschlossen bzw. geöffnet werden. Eine derartige Positioniereinrichtung erweist sich bezüglich Verschmutzungen als anfällig, was die Funktionsweise des Analysegerätes beeinträchtigt und zu Fehlfunktionen führt. Diese sind typischerweise durch ein kontinuierliches Entnehmen von Testelementen aus einem Vorratsbehälter bis zu dessen vollständiger Leerung (Dauerstreifenschieber) oder durch ein Verklemmen von Testelementen in dem Förderweg aufgrund eines Übereinanderschiebens von Testelementen (Streifenklemmer) geprägt.

Analysehandgeräte zum Untersuchen eines medizinisch bedeutsamen Bestandteils einer Probe, wie beispielsweise Geräte zum Bestimmen des Blutglucosegehaltes, werden häufig von Personen verwendet, deren Wahrnehmung oder manuelle Geschicklichkeit wegen Krankheit oder Alter eingeschränkt ist. Es ist deshalb wichtig, dass sich solche Analysegeräte möglichst leicht handhaben lassen und Fehlbedienungen oder Fehlfunktionen möglichst ausgeschlossen sind.

Der Erfindung liegt die Aufgabe zugrunde, ein Analysehandgerät zum Untersuchen eines medizinisch bedeutsamen Bestandteils einer Probe und ein Verfahren zum Betreiben eines solchen Analysehandgeräts anzugeben, das Fehlfunktionen oder damit verbundene Fehlbedienungen reduziert.

Diese Aufgabe wird erfindungsgemäß durch ein Analysehandgerät bzw. ein Verfahren zum Betreiben eines solchen mit den Merkmalen der beigefügten unabhängigen Patentansprüche gelöst. Bevorzugte Ausgestaltungen und Weiterbildungen der Erfindung ergeben sich aus den abhängigen Patentansprüchen und der nachfolgenden Beschreibung mit zugehörigen Zeichnungen.

Ein erfindungsgemäßes Analysehandgerät weist also die Besonderheit auf, dass es eine Prüfeinheit umfasst, die eine korrekte Positionierung eines analytischen Verbrauchsmittels im Förderweg feststellt, wobei die Prüfeinheit sowohl eine elektrische Schaltkomponente, die eine Positionierung eines analytischen Verbrauchsmittels auf dem Förderweg mechanisch abtastet, die wenigstens eine Schaltposition einnimmt, die das Vorhandensein eines analytischen Verbrauchsmittels repräsentiert, und die in Abhängigkeit von der Positionierung des analytischen Verbrauchsmittels ein Schaltsignal abgibt, als auch eine optische Sensoreinheit, die eine Positionierung eines analytischen Verbrauchsmittels auf dem Förderweg optisch abtastet und die in Abhängigkeit von der Positionierung des analytischen Verbrauchsmittels ein Sensorsignal abgibt, und eine Steuereinheit aufweist, die das Schaltsignal der elektrischen Schaltkomponente und das Sensorsignal der optischen Sensoreinheit auswertet und in Abhängigkeit von dem Vergleich dieser Signale das Analysehandgerät ansteuert.

Bei dem erfindungsgemäßen Analysehandgerät ist eine Prüfeinheit vorgesehen, die eine korrekte Positionierung eines analytischen Verbrauchsmittels im Förderweg feststellt. Unter einer Positionierung eines Verbrauchsmittels im Förderweg wird dabei eine Situation verstanden, in der die Entnahmeeinrichtung das Entnehmen eines Verbrauchsmittels aus dem Magazin und das Fördern des Verbrauchsmittels auf den Förderweg durchgeführt hat, so dass sich also, wenn die Entnahme aus dem Magazin fehlerfrei bzw. erfolgreich war, ein Verbrauchsmittel im Förderweg befindet, also im Förderweg positioniert ist. Unter korrekter Positionierung wird dabei verstanden, dass das Verbrauchsmittel sich tatsächlich in einer durch den Verfahrensablauf vorgegebenen Position befindet. In einer solchen vorgegebenen Position wird beispielsweise das bloße Vorhandensein des Verbrauchsmittels festgestellt, also überprüft, ob es an sich im Förderweg vorhanden und insoweit korrekt positioniert ist, als es an dem vorgesehenen Ort oder Bereich vorhanden ist. In anderen Fällen kann auch eine genaue Positionsüberprüfung des Verbrauchsmittels erfolgen, wobei festgestellt wird, ob sich das Verbrauchsmittel in einer exakten Lage bzw. an einer exakten Position auf dem Förderweg befindet. Die Überprüfung der korrekten Positionierung des Verbrauchsmittels kann sich in diesen Fällen auf eine beliebige Position des Verbrauchsmittels im Förderweg beziehen. In manchen Ausführungsformen kann die Überprüfung der korrekten Positionierung des Verbrauchsmittels in der Position erfolgen, in der mit dem Analysesensor die Analyse durchgeführt wird. In anderen Ausführungsformen kann es aber auch vorteilhaft sein, die korrekte Positionierung des Verbrauchsmittels in einer anderen Position als dieser Messposition zu überprüfen, insbesondere in einer Position, die von dem Verbrauchsmittel vor dem Erreichen der Messposition erreicht wird.

Die Prüfeinheit umfasst eine elektrische Schaltkomponente und zusätzlich eine optische Sensoreinheit, die jede für sich und gemeinsam eine Positionierung des analytischen Verbrauchsmittels auf dem Förderweg abtasten und entsprechend der abgetasteten Positionierung ein Schaltsignal bzw. Sensorsignal abgeben, die gemeinsam von einer Steuereinheit dahingehend ausgewertet werden, dass eine Verifizierung bzw. Überprüfung eines Signals anhand des anderen Signals ermöglicht ist. Dadurch gelingt es, Störungen oder Fehlfunktionen, insbesondere der elektrischen Schaltkomponente, die sich insbesondere aufgrund von Verschmutzungen der elektrischen Kontakte ergeben, zielgerichtet zu überprüfen und einen daraus resultierenden Schaden zu begrenzen.

Beispielsweise gelingt es durch die erfindungsgemäße Ausbildung des Analysehandgerätes sicherzustellen, dass Fehlfunktionen aufgrund verschmutzter elektrischer Kontakte der elektrischen Schaltkomponente erheblich eingeschränkt sind. Derartige Verschmutzungen treten gerade bei der mobilen Verwendung der Analysehandgeräte im besonderen Maße auf, da diese Geräte durch die Benutzer in den unterschiedlichsten Situationen und insbesondere in wenig geeigneten Umgebungen, die sich durch besondere Feuchtigkeit und Verschmutzung auszeichnen, benutzt bzw. gelagert werden. Das Risiko einer Verschmutzung der Kontakte ist insbesondere dadurch gegeben, dass das Gehäuse des Analysehandgerätes Öffnungen, insbesondere zum Zuführen oder Entnehmen der analytischen Verbrauchsmittel, aufweist, durch die Staub und andere Schmutzpartikel in das Gehäuse eindringen und sich unter anderem auf die Kontakte der elektrischen Schaltkomponente absetzen und Einfluss auf das elektrische Schaltverhalten nehmen können. Auch eine Beeinträchtigung der mechanischen Funktionsfähigkeit der elektrischen Schaltkomponente kann auftreten.

Beispielsweise kann dies zu Fehlfunktionen führen, die als Dauerstreifenschieber oder als Streifenklemmer bezeichnet werden. Bei dem Dauerstreifenschieber wird durch die Steuereinheit der durch die Verschmutzung verhinderte elektrische Kontakt als nicht vorhandenes Verbrauchsmittel interpretiert, so dass aus dem Magazin mit Hilfe der Entnahmeeinrichtung ein neues Verbrauchsmittel entnommen und auf den Förderweg gefördert wird, bis es in den Bereich des elektrischen Schaltmittels gelangt. Dies wiederholt sich, bis entweder das Magazin vollständig entleert ist (Dauerstreifenschieber) oder bis sich die Verbrauchsmittel übereinander geschoben und im Förderweg verkeilt haben (Streifenklemmer) und dadurch die Funktionsfähigkeit des Analysehandgerätes ausgeschlossen ist. Gerade diese beschriebenen, für den Benutzer sehr ärgerlichen und durch den unnötigen Verbrauch an Verbrauchsmitteln kostspieligen Fehlfunktionen lassen sich erfindungsgemäß deutlich reduzieren.

Durch die erfindungsgemäße Realisierung des Analysehandgerätes mit der erfindungsgemäßen Prüfeinheit, die eine Verifizierung bzw. Überprüfung des Schaltsignals der elektrischen Schaltkomponente anhand des optischen Sensorsignals der optischen Sensoreinheit ermöglicht, wird durch die optische Sensoreinheit eine Überprüfung des elektrischen Schaltsignals insbesondere mit dem verifizierten Ergebnis, dass kein Verbrauchsmittel im Förderweg vorhanden ist, ermöglicht. Wird dabei von der optischen Sensoreinheit im Gegensatz zu dem unrichtigen Signal der defekten elektrischen Schaltkomponente das Vorhandensein des Verbrauchsmittels beziehungsweise dessen korrekte Positionierung detektiert bzw. festgestellt, so wird die Steuereinheit das kontinuierliche weitere Entnehmen der Verbrauchsmittel aus dem Magazin mit Hilfe der Entnahmeeinrichtung verhindern, wodurch die vorgenannten Fehlfunktionen weitgehend ausgeschlossen sind.

Durch die erfindungsgemäße Ausbildung der Prüfeinheit mit einer elektrischen Schaltkomponente, die eine korrekte Positionierung eines analytischen Verbrauchsmittels auf Basis eines mechanischen Abtastvorganges ermittelt, und einer optischen Sensoreinheit, die eine korrekte Positionierung auf Basis einer optischen Abtastung ermittelt, ist ein sehr universelles und sicheres Überprüfen einer korrekten Positionierung durch die Prüfeinheit gegeben.

Vorzugsweise wird die elektrische Schaltkomponente so ausgebildet, dass sie ein verschiebliches Schaltelement enthält, das beispielhaft als federbewehrter, verschiebbarer Zapfen realisiert ist, und das dazu verwendet wird, die Oberfläche des Verbrauchsmittels, das typischerweise mit streifenförmiger Gestalt oder als im wesentlichen quadratisches Blättchen ausgebildet ist, mechanisch abzutasten und über eine Verschiebung des Schaltelements als Folge des Abtastvorganges einen elektrischen Kontakt zu schließen oder zu öffnen. Durch das mechanische Abtasten der Oberfläche des Verbrauchsmittels gelingt es sehr verlässlich und auf robuste Weise eine Information über die Positionierung des Verbrauchsmittels im Bereich der elektrischen Schaltkomponente zu gewinnen. Gerade diese elektrische Schaltkomponente erweist sich als wenig anfällig gegen Verformungen des Verbrauchsmittels oder Erschütterungen des Analysehandgerätes.

Durch die erfindungsgemäße Kombination dieser robusten, wenig anfälligen elektrischen Schaltkomponente zum Erfassen einer korrekten Positionierung mit der optischen Sensoreinheit, die die korrekte Positionierung des Verbrauchsmaterials aufgrund einer optischen Erfassung feststellt, ist die Verlässlichkeit der Information der Prüfeinheit zur korrekten Positionierung wesentlich erhöht. Dabei wird die optische Sensoreinheit entweder als passive optische Sensoreinheit oder als aktive optische Sensoreinheit realisiert. Die passive optische Sensoreinheit enthält ausschließlich einen optischen Empfänger, der charakteristische optische Eigenschaften des Verbrauchsmittels im Sensorbereich der optischen Sensoreinheit, der sich im Förderweg befindet, erfasst. Aktive optische Sensoreinheiten zeichnen sich durch eine aktive Beleuchtung des Sensorbereiches aus, so dass aktive optische Sensoreinheiten auch unter schwierigen Umständen ein verlässliches Sensorsignal erzeugen.

Gerade im Fall von Verschmutzungen auf dem Verbrauchsmittel zeigen aktive optische Sensoreinheiten eine merklich verbesserte Signalqualität. Dabei wird die optische Sensoreinheit bevorzugt im reflektierenden Betrieb verwendet, so dass sich die aktive Beleuchtung des Sensorbereiches auf derselben Seite des Förderwegs befindet wie der optische Empfänger, der die vom Verbrauchsmittel reflektierte Strahlung erfasst. Hierdurch ist eine sehr kompakte Ausbildung der optischen Sensoreinheit ermöglicht. Alternativ hierzu hat sich auch die transmittierende Ausbildung des optischen Sensors bewährt, bei der die einzelnen Komponenten zu beiden Seiten des Förderwegs bzw. des Verbrauchsmittels und damit einander gegenüberliegend angeordnet sind. Dies ermöglicht eine getrennte Ausbildung der einzelnen Komponenten der optischen Sensoreinheit, die jeweils für sich einen kleineren Bauraum bei erhöhtem Verkabelungsaufwand benötigen und dadurch für den Einbau in einem sehr kleinen Analysehandgerät besonders vorteilhaft sind.

Durch die bevorzugte Ausbildung der elektrischen Schaltkomponente mit einem verschiebbaren zapfenförmigen Schaltelement, das ein dem analytischen Verbrauchsmittel zugewandtes, in Richtung des Verbrauchsmittels konisch zulaufendes Ende aufweist, gelingt es, nicht nur das Vorhandensein bzw. die Positionierung des analytischen Verbrauchsmittels im Bereich des Schaltelements festzustellen, sondern zusätzlich das Verbrauchsmittel aktiv zu positionieren, indem der Zapfen mit seinem konisch zulaufenden Ende in eine Erhebung und/oder Ausnehmung des Verbrauchsmittels so eingreift, dass das Verbrauchsmittel in eine gewünschte Analyseposition verschoben bzw. in dieser gehalten wird. Unter Ausnehmung wird auch eine Öffnung oder ein Loch in dem Verbrauchsmittel verstanden. Zur Unterstützung dieser Positionierfunktion wird der verschiebbare Zapfen vorzugsweise dahingehend federnd gelagert, dass die Federwirkung auf das Verbrauchsmittel einwirkt und dieses in die gewünschte Analyseposition bewegt.

Durch diese kombinierte Funktion der Prüfeinheit einerseits als doppelte und damit verifizierende bzw. überprüfende Einheit zum Bestimmen einer korrekten Positionierung des analytischen Verbrauchsmittels und andererseits als Einheit zum aktiven Positionieren in der gewünschten Analyseposition ist eine sehr verlässliche und wenig anfällige und damit fehlertolerante Realisierung eines Analysehandgerätes geschaffen. Diese durch die Positionierung eingenommene Position für die potentielle Analyse stellt eine optimale Position zur optischen Erfassung der korrekten Positionierung des Verbrauchsmittels dar, was einen sehr sicheren Betrieb des Analysehandgerätes ermöglicht. Die elektrische Schaltkomponente kann dabei so ausgebildet sein, dass sich ihre Prüffunktion und ihre Positionierfunktion auf die gleiche oder auf unterschiedliche Positionen des analytischen Verbrauchsmittels in dem Förderweg beziehen.

Durch die bevorzugte Ausbildung des Analysehandgerätes mit einer elektrischen Schaltkomponente, die mit einer positionsspezifischen Oberflächengestaltung des analytischen Verbrauchsmittels zusammenwirkt, und die abhängig von der Lage des verschieblichen Schaltelementes unterschiedliche Schaltzustände realisiert, gelingt es, ein sehr differenziertes Schaltverhalten der Prüfeinheit zu ermöglichen. Dabei ist diese positionsspezifische Oberflächengestaltung durch eine Kontur realisiert, die sich insbesondere durch eine in Breite und/oder in Tiefe variierende Rinne oder durch eine in Breite und/oder Höhe variierende Rampe auf dem analytischen Verbrauchsmittel auszeichnet. Durch das Eindringen des Schaltelementes in die positionsdifferenzierende Rinne bzw. das Abheben aufgrund der positionsdifferenzierenden Rampe gelingt es, unterschiedliche Positionen des Verbrauchsmittels selektiv zu erfassen und das Analysehandgerät mittels der Steuereinheit positionsdifferenziert zu steuern. Insbesondere wird es möglich, den Zeitpunkt der Analyse optimal zu steuern. Gerade im Hinblick auf eine verbrauchseffiziente Betriebsweise erweist sich dies als besonders vorteilhaft, da die einzelnen elektrischen Komponenten des Analysehandgerätes zielgerichtet frühzeitig aktiviert bzw. deaktiviert werden können, um den elektrischen Energieverbrauch möglichst gering zu halten. Hierdurch gelingt es, die Häufigkeit von Fehlfunktionen zu reduzieren.

Eine besonders bevorzugte Ausbildung des Analysehandgerätes ist dadurch ausgezeichnet, dass die optische Sensoreinheit so ausgebildet ist, dass sie die Position eines Schaltelementes der elektrischen Schaltkomponente, beispielsweise eines verschiebbaren Zapfens, selbst erfasst und damit die Möglichkeit schafft, das elektrische Schaltsignal der elektrischen Schaltkomponente in Relation zu der Position des Schaltelementes, das von der Positionierung des analytischen Verbrauchsmaterials im Förderweg abhängt, zu setzen und dadurch eine Verifizierung bzw. Überprüfung des Positionierungssignals der elektrischen Schaltkomponente mittels der optischen Sensoreinheit zu ermöglichen. Diese Ausbildung der Prüfeinheit ermöglicht einen sehr kompakten Aufbau, der eine Modularisierung der Prüfeinheit in einem Analysehandgerät schafft. Die hierdurch gewonnene gute Integrationsmöglichkeit der Prüfeinheit schafft zudem eine reparaturfreundliche Realisierung des erfindungsgemäßen Analysehandgerätes.

Nach einer bevorzugten Ausbildung des Analysehandgerätes wird die optische Sensoreinheit zum Erfassen einer Positionierung des analytischen Verbrauchsmittels auf dem Förderweg mit dem Analysesensor in einer gemeinsamen Sensoreinheit integriert. Die Integration kann dabei bedeuten, dass beide Sensoren in einer Baueinheit zusammengefasst werden, oder bevorzugt bedeuten, dass beide Sensoren in einem einzigen Sensor, der beide Funktionen erfüllt, realisiert werden. Gerade bei der Ausbildung beider Sensoren in Form optischer Sensoren wird es bevorzugt möglich, mit einem einzigen Sensor beide Funktionalitäten gemeinsam zu realisieren. In diesem Fall werden die beiden Funktionalitäten zeitlich aufeinander folgend realisiert, zuerst die Funktionalität der optischen Sensoreinheit zum Erfassen der Positionierung des analytischen Verbrauchsmittels auf dem Förderweg und danach die Funktionalität des optischen Analysesensors für die optische Analyse der Probe. Dieses erfindungsgemäße Analysehandgerät zeigt neben der Reduktion von Fehlfunktionen einen sehr kompakten Aufbau, der die Handhabbarkeit des Analysehandgerätes besonders fördert. Darüber hinaus ist aufgrund der Reduktion der Sensoren eine Verbesserung der Zuverlässigkeit und damit eine zusätzliche Reduktion von Fehlfunktionen gegeben.

Nach einer bevorzugten Ausbildung des Analysehandgerätes wird das Entnehmen eines analytischen Verbrauchsmittels aus dem Magazin durch die Entnahmeeinrichtung dann mittels der Steuereinheit verhindert, wenn entweder das Schaltsignal der elektrischen Schaltkomponente oder das Sensorsignal der optischen Sensoreinheit eine korrekte Positionierung und das jeweils andere Signal eine nicht korrekte Positionierung eines analytischen Verbrauchsmittels auf dem Förderweg repräsentiert und somit widersprüchliche Signale vorliegen. Damit wird sichergestellt, dass ein Sensorsignal, das den Zustand "nicht korrekte Positionierung des Verbrauchsmittels" repräsentiert und damit auch das nicht Vorhandensein eines Verbrauchsmittels bedeuten kann, nicht zum Entnehmen eines neuen Verbrauchsmittels aus dem Magazin führt und sich damit kein unnötiger Verbrauch an Verbrauchsmitteln ergibt, wodurch somit die Fehlfunktion des sogenannten Dauerstreifenschiebers erfindungsgemäß verhindert wird. Auch die Gefahr eines Verkeilens mehrerer analytischer Verbrauchsmitteln im Förderweg (so genannter Streifenklemmer) ist durch diese Ausbildung des Analysehandgerätes mit dieser Funktionalität weitgehend beseitigt. Damit gelingt es, die Anzahl an Fehlfunktionen durch die erfindungsgemäße Ausbildung des Analysehandgerätes zu reduzieren.

Als besonders vorteilhaft hat es sich erwiesen, den Analysesensor zum Untersuchen einer Probe auf einen medizinisch bedeutsamen Bestandteil dann zu aktivieren und damit die Analyse zu bewirken, wenn mittels eines Schaltsignals der elektrischen Schaltkomponente, das eine korrekte Positionierung eines analytischen Verbrauchsmittels repräsentiert, eine korrekte Positionierung eines analytischen Verbrauchsmittels im Förderweg festgestellt wurde. Vorzugsweise wird dies unabhängig von dem optischen Sensorsignal realisiert, was zu einer sehr einfachen und robusten Realisierung des Analysehandgerätes und des Verfahrens zum Betreiben des Analysehandgerätes führt. Die elektrische Schaltkomponente ist dabei gewissermaßen gegenüber der optischen Sensoreinheit priorisiert und die optische Sensoreinheit tritt nur dann wirksam zum Überprüfen bzw. Verifizieren des Schaltsignals der elektrischen Schaltkomponente in Funktion, wenn das Schaltsignal der elektrischen Schaltkomponente angibt, es sei kein analytisches Verbrauchsmittel in der korrekten Position auf dem Förderweg.

Die Erfindung bezieht sich weiterhin auf ein Verfahren zum Betreiben eines Analysehandgerätes zum Untersuchen einer Probe, insbesondere einer biologischen Flüssigkeit, auf einen medizinisch bedeutsamen Bestandteil, wobei das Analysehandgerät eine Anzeigeeinrichtung, eine Entnahmeeinrichtung zum Entnehmen eines analytischen Verbrauchsmittels aus einem Magazin, insbesondere einem Trommelmagazin, und zum Fördern auf einen Förderweg, einen Analysesensor, dem auf dem Förderweg ein analytisches Verbrauchsmittel zuführbar ist, und eine Prüfeinheit, die eine korrekte Positionierung eines analytischen Verbrauchsmittels im Förderweg feststellt, umfasst, und das die Besonderheit aufweist, dass nach einer Aktivierung des Analysehandgeräts und der darauf folgenden Entnahme eines analytischen Verbrauchsmittels aus dem Magazin und dem Fördern auf den Förderweg Signale einer elektrischen Schaltkomponente, die eine Positionierung eines analytischen Verbrauchsmittels auf dem Förderweg mechanisch abtastet, und einer optischen Sensoreinheit, die eine Positionierung eines analytischen Verbrauchsmittels auf dem Förderweg optisch abtastet, mittels einer Steuereinheit ausgewertet werden und in Abhängigkeit von dem Vergleich dieser Signale das Analysehandgerät angesteuert wird.

Dabei wird die gemeinsame Auswertung der Signale dahingehend vorgenommen, dass eine Verifizierung bzw. Überprüfung der jeweils erfassten Positionierungen vorgenommen wird und abhängig davon werden insbesondere der Analysesensor bzw. die Entnahmeeinrichtung und/oder die Anzeigeeinrichtung angesteuert. Durch die erfindungsgemäße Möglichkeit der Überprüfung bzw. Verifizierung der verschiedenen Signale, die eine Positionierung des Verbrauchsmittels auf dem Förderweg repräsentieren, gelingt es, Fehlfunktionen und ggf. damit verbundene Fehlbedienungen zu reduzieren, was die Einsetzbarkeit und Gebrauchszuverlässigkeit des erfindungsgemäßen Analysegerätes wesentlich verbessert.

Als besonders vorteilhaft hat es sich erwiesen, unabhängig von dem optischen Sensorsignal den Messvorgang mit dem Analysesensor zu starten oder freizugeben, wenn durch die elektrische Schaltkomponente eine korrekte Positionierung eines analytischen Verbrauchsmittels festgestellt wurde. Diese Ausbildung macht es sich zu Nutzen, dass das Schaltsignal der elektrischen Schaltkomponente, das positiv eine korrekte Positionierung repräsentiert, ein sehr verlässliches Signal darstellt, das im Regelfall keiner Überprüfung bzw. Verifizierung bedarf. Durch diese Ausbildung ist ein sehr einfaches und ausreichend verlässliches Verfahren zum Betreiben eines Analysehandgerätes geschaffen.

Es hat sich als vorteilhaft erwiesen, das Verfahren zum Betreiben des Analysehandgerätes dahingehend auszubilden, dass das Entnehmen eines analytischen Verbrauchsmittels durch die Entnahmeeinrichtung unterdrückt wird, wenn entweder das Signal der elektrischen Schaltkomponente oder der optischen Sensoreinheit eine korrekte Positionierung und das jeweils andere Signal eine nicht korrekte Positionierung eines analytischen Verbrauchsmittels repräsentiert, so dass sich die beiden Signale widersprechen. In diesem Falle ist erfindungsgemäß sichergestellt, dass das Entnehmen eines weiteren neuen analytischen Verbrauchsmittels aus dem Magazin verhindert wird, so dass die unerwünschten Fehlfunktionen einer fortlaufenden Förderung von Verbrauchsmitteln in der Art eines Dauerstreifenschiebers bzw. eines Verkeilens von Verbrauchsmitteln im Förderweg in der Art eines Streifenklemmers verhindert wird. Vorzugsweise erfolgt zusätzlich eine spezifische Fehlermeldung mit Hilfe der Anzeigeeinrichtung.

Wird sowohl von der elektrischen Schaltkomponente wie auch von der optischen Sensoreinheit eine nicht korrekte Positionierung des analytischen Verbrauchsmittels, insbesondere das nicht Vorhandensein eines solchen festgestellt und damit wechselseitig bestätigt, so wird die Entnahmeeinrichtung aktiviert und damit ein Verbrauchsmittel aus dem Magazin entnommen und über den Förderweg in Richtung Analysesensor gefördert, wo mit Hilfe der erfindungsgemäßen Prüfeinheit eine korrekte Positionierung des Verbrauchsmittels erfasst und überprüft wird. Durch diese erfindungsgemäße Art der Überprüfung der korrekten Positionierung des Verbrauchsmittels auf dem Förderweg ist ein sehr sicherer Betrieb des Analysehandgerätes gegeben, der sich durch eine sehr geringe Anzahl an Fehlfunktion auszeichnet.

Die Betriebssicherheit lässt sich weiter verbessern, indem überprüft wird, ob eine vollständige Entleerung des Magazins gegeben ist, und in diesem Falle eine weitere Aktivierung der Entnahmeeinrichtung bei Feststellung einer nicht korrekten Positionierung und der zugehörigen Bestätigung verhindert wird. Vorzugsweise wird mit Hilfe der Anzeigeeinrichtung eine Fehlermeldung ausgegeben, die auf das vollständig entleerte Magazin hinweist. Durch diese Ausbildung des erfindungsgemäßen Verfahrens ist eine besonders angenehme Handhabung des Analysegerätes gegeben, die eine Fehlbedienung des Analysehandgerätes oder eine Fehlfunktion desselben weitgehend ausschließt.

Weitere Einzelheiten und Vorteile der Erfindung werden anhand von Ausführungsbeispielen unter Bezugnahme auf die beigefügten Zeichnungen erläutert. Die beschriebenen Merkmale können einzeln oder in Kombination verwendet werden, um bevorzugte Ausgestaltungen der Erfindung zu schaffen.

Es zeigen:
- Fig. 1: ein Ausführungsbeispiel eines Analysehandgerätes;
- Fig. 2: das gezeigte Analysehandgerät von Fig. 1 bei geöffnetem Magazinfach mit Trommelmagazin und analytischem Verbrauchsmittel;
- Fig. 3: eine weitere Ansicht des Analysehandgeräts von Fig. 2;
- Fig. 4: einen Ausschnitt eines Ausführungsbeispiels eines Analyse-handgerätes mit elektrischer Schaltkomponente und optischer Sensoreinheit; und
- Fig. 5: ein Diagramm eines beispielhaften Verfahrens zum Betreiben eines Analysehandgerätes.

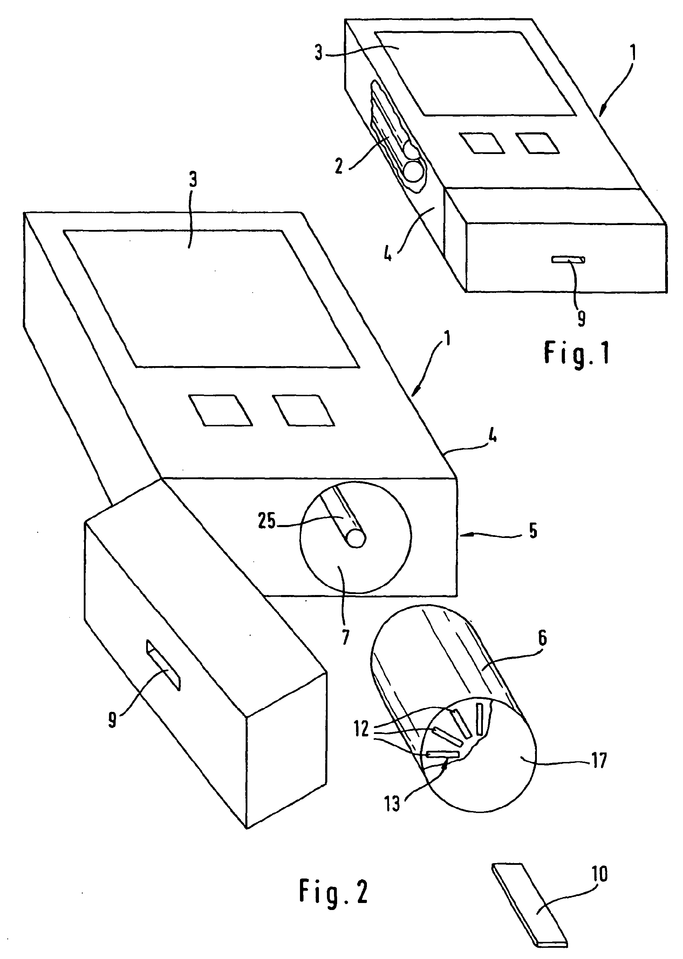

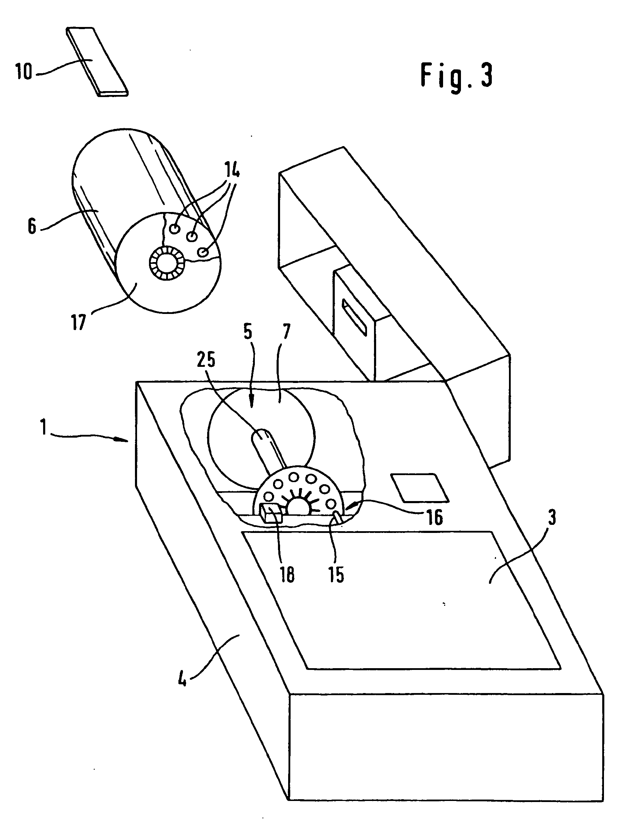

Fig. 1 bis 3 zeigen in verschiedenen Ansichten ein kompaktes, tragbares Analysehandgerät 1 zum Untersuchen eines medizinisch bedeutsamen Bestandteils einer Probe, insbesondere einer biologischen Flüssigkeit, wie beispielsweise Blut, Urin oder Speichel. Das in Fig. 1 gezeigte Analysehandgerät 1 dient zum Bestimmen des Blutglucosegehaltes und weist eine integrierte Stromquelle 2 in Form handelsüblicher Batterien oder Solarzellen auf. Das Ergebnis einer Untersuchung wird mit einer Anzeigeeinrichtung 3, bevorzugt einem stromsparenden Flüssigkristalldisplay oder einem OLED-Display angezeigt. Das Analysehandgerät 1 weist ein Gehäuse 4 auf, das eine Ladeöffnung 5 zum Aufnehmen eines auswechselbaren Trommelmagazins 6 in ein Magazinfach 7 hat, in dem das Trommelmagazin 6 mittels eines Antriebs schrittweise um seine geometrische Längsachse rotierbar ist. Fig. 1 zeigt das Analysehandgerät 1 mit geschlossener Ladeöffnung 5. Fig. 2 und 3 zeigen das Analysehandgerät 1 bei geöffneter Ladeöffnung 5. In Fig. 3 ist zur besseren Veranschaulichung ein Teil des Gehäuses 4 durchbrochen dargestellt, so dass das Magazinfach 7 einsehbar ist.

In einer Stirnseite weist das Gehäuse 4 eine Ausgabeöffnung 9 für in dem Trommelmagazin 6 gelagerte analytische Verbrauchsmittel 10 auf. Bevorzugt sind diese Verbrauchsmittel 10 als Teststreifen ausgebildet, auf die eine Probe aufgebracht werden kann. Ein im Teststreifen enthaltenes Reagenz reagiert mit einem medizinisch bedeutsamen Bestandteil der Probe, so dass das Ergebnis der Reaktion mit einer Analyseeinrichtung des Analysehandgerätes 1 ausgewertet werden kann. Eine solche Analyseeinrichtung kann beispielsweise einen optischen Sensor als Analysesensor enthalten, der eine Farbänderung eines als Teststreifen ausgebildeten Verbrauchsmittels 10 erfasst, oder einen Sensor enthalten, der eine Leitfähigkeitsänderung der Probe bestimmt.

Das Trommelmagazin 6 hat mehrere ringförmig um seine geometrische Längsachse angeordnete Kammern 12, die analytische Verbrauchsmittel 10 enthalten können. Durch schrittweise Rotation des Trommelmagazins 6 können die Kammern 12 nacheinander in einer Entnahmeposition positioniert werden, so dass die Verbrauchsmittel 10 bei Bedarf aus der jeweiligen Kammer 12 des Trommelmagazins 6 entnommen und durch die Ausgabeöffnung 9 des Gehäuses 4 ausgegeben werden können.

Die Zahl dieser Kammern 12 kann weitgehend beliebig gewählt werden. In der Regel sind 10 bis 100 Kammern 12 zweckmäßig, bevorzugt sind 15 bis 30 Kammern 12 vorhanden. Jede der Kammern 12 weist an einer Stirnseite des Trommelmagazins 6 eine Entnahmeöffnung 13 zum Entnehmen eines Verbrauchsmittels 10 und eine der Entnahmeöffnung 13 gegenüberliegende Einschuböffnung 14 zum Einführen eines Stößels 15 einer Entnahmeeinrichtung 16 auf. Die Einschuböffnungen 14 und die Entnahmeöffnungen 13 sind zum Schutz der Verbrauchsmittel 10 mit einer Siegelfolie 17 verschlossen. Wie in der EP 1 022 565 A2 beschrieben lassen sich mit dem Stößel 15 Verbrauchsmittel 10 zur Verwendung aus den Kammern 12 herausschieben, wobei die Siegelfolie 17 der Einschuböffnung 14 vom Stößel 15 und die Siegelfolie 17 der Entnahmeöffnung 13 von dem Verbrauchsmittel 10 durchstoßen wird.

Mit Hilfe der Trommelprüfeinrichtung 18 wird ein Signal erzeugt, das eine Information darüber enthält, ob eine der Einschuböffnungen 14 mit Siegelfolie 17 verschlossen ist und damit ein zur Verwendung vorgesehenes Verbrauchsmittel 10 darin enthalten ist. Mittels der Trommelprüfvorrichtung 18 lässt sich feststellen, ob das Trommelmagazin 6 vollständig entleert ist. Alternativ oder zusätzlich zu der Trommelprüfeinrichtung 18 kann auch eine Zähleinrichtung vorgesehen sein, welche die Anzahl der aus den Kammern 12 eines Trommelmagazins 6 entnommenen Verbrauchsmittel zählt und bei Erreichen der Maximalzahl ein "Trommel leer"-Signal ausgibt.

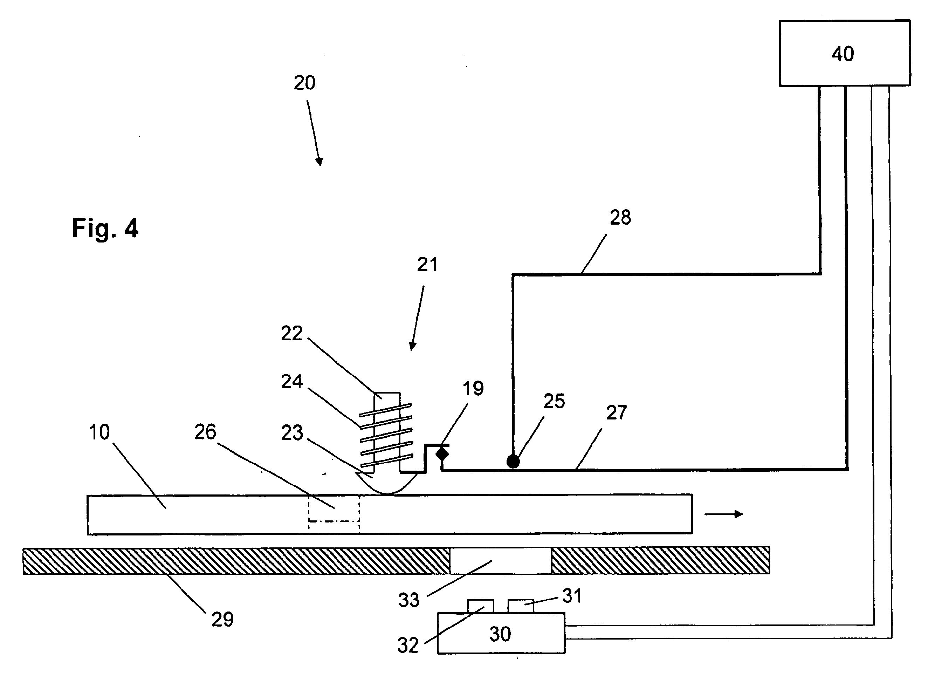

Fig. 4 zeigt in einer schematischen Darstellung einen Aufbau der Prüfeinheit 20, die eine korrekte Positionierung eines analytischen Verbrauchsmittels 10 im Förderweg feststellt und dabei zusätzlich als Positioniereinrichtung eine korrekte Positionierung unterstützt bzw. eine korrekte Positionierung bewahrt.

Die Prüfeinheit 20 besteht im Wesentlichen aus der Steuereinheit 40 und der optischen Sensoreinheit 30 sowie der elektrischen Schaltkomponente 21. Sowohl die optische Sensoreinheit 30 als auch die elektrische Schaltkomponente 21 haben die Aufgabe, eine Positionierung eines analytischen Verbrauchsmittels 10 auf dem Förderweg abzutasten und ein die Positionierung repräsentierendes Schaltsignal bzw. ein entsprechendes Sensorsignal an die Steuereinheit 40 abzugeben, um auf Basis einer gemeinsamen Auswertung dieser Signale das Analysehandgerät 1 bzw. einzelne Komponenten davon zu steuern.

Die elektrische Schaltkomponente 21 weist einen verschiebbar ausgebildeten Zapfen 22 mit einem dem analytischen Verbrauchsmittel 10 zugeordneten Ende 23 auf, das in Richtung des Verbrauchsmittels 10 konisch zuläuft. Das konisch zulaufende Ende 23 des Zapfens 22 ist so ausgebildet, dass es in der Messposition des Verbrauchsmittels 10 in eine Ausnehmung 26 des analytischen Verbrauchsmittels 10 eingreifen und dieses aufgrund des konischen Verlaufes des Endes 23 positionieren kann. Durch diese Positionierung im Sinne einer Lagefixierung ist eine sichere optische Analyse des Verbrauchsmittels 10 mittels des Analysesensors des Analysehandgerätes 1, der beispielsweise auch durch die optische Sensoreinheit 30 realisiert wird, gewährleistet. Eine sichere optische Erfassung einer korrekten Positionierung des Verbrauchsmittels 10 mit Hilfe der optischen Sensoreinheit 30 kann dadurch ebenfalls gewährleistet werden. Die Ausnehmung 26 kann auch als das Verbrauchsmittel 10 durchdringendes Loch ausgebildet sein; dies ist in Fig. 4 durch einen punkt-strichlierten Querstrich in der Ausnehmung 26 angedeutet. Weitere vorteilhafte Ausführungsformen elektrischer Schaltkomponenten 21 sind in dem Dokument EP 1 508 807 A2 beschrieben.

In der Fig. 4 ist das Verbrauchsmittel 10 jedoch noch nicht in der Messposition im Förderweg angeordnet, in der die Analyse durchgeführt wird. Demzufolge ist das konisch zulaufende Ende 23 des Zapfens 22 auch noch nicht in die Ausnehmung 26 bzw. das Loch in dem Verbrauchsmittels 10 eingedrungen und liegt auf der Oberfläche des Verbrauchsmittels 10 auf. Das Verbrauchsmittel 10 wurde durch die Pfeilrichtung in den Förderweg bis zu der dargestellten Position gefördert, in der seine korrekte Positionierung im Förderweg mittels der Prüfeinheit 20 überprüft wird. Bei dieser Überprüfung wird geprüft, ob sich ein Verbrauchsmittel 10 in der Prüfposition im Förderweg befindet, es also sozusagen aus einer Kammer des Trommelmagazins durch das Fördern mittels der Entnahmeeinrichtung in dieser Prüfposition angekommen ist. Nach erfolgter Prüfung der korrekten Positionierung des Verbrauchsmittels 10 (in der dargestellen Prüfposition) wird es in Pfeilrichtung weitergefördert, bis es die Messposition erreicht, in der das Ende 23 in die Ausnehmung 26 eindringt und die Analyse durchgeführt wird.

Das Verbrauchsmittel 10 ist im Förderweg im Bereich der elektrischen Schaltkomponente 21 bzw. im Bereich der optischen Sensoreinheit 30 auf einer Auflagefläche 29 liegend angeordnet. Durch das Einbringen des analytischen Verbrauchsmittels 10 ist der Zapfen 22 aus der Ruheposition, die durch eine Berührung des Endes 23 auf der Auflagefläche 29 festgelegt ist, nach oben in die dargestellte Position verschoben, in der er auf der Oberseite des Verbrauchsmittels 10 aufliegt. Diese Verschiebung wird durch den Vorschub des Verbrauchsmittels 10 in Pfeilrichtung und das Entlanggleiten entlang der Ablaufkante des konisch zulaufenden Endes 23 bewirkt. Diese Verschiebung des Zapfens 22 senkrecht zum Förderweg bzw. zur Auflagefläche 29 erfolgt entgegen einer Federkraft, die durch die Feder 24 erzeugt wird. Die Feder 24 stellt sicher, dass der Zapfen 22 in der Ruheposition gehalten wird, bis das Verbrauchsmittel 10 ihn entgegen der Federkraft in eine verschobene, beispielsweise in die dargestellte Position verschiebt.

Abhängig von der Verschiebung des Zapfens 22 wird mittels des Zapfens 22 und einem mechanischen Kontakt 19 zwischen dem Zapfen 22 und einer Kontaktfeder 27 ein elektrischer Kontakt 25 geschlossen oder geöffnet. Die beiden Kontakte dieses elektrischen Kontakts 25 sind durch die bewegliche Kontaktfeder 27 und die feste Federplatte 28 realisiert. Der Zapfen 22 ermöglicht in der dargestellten Situation einen Kontakt zwischen Kontaktfeder 27 und Federplatte 28 zum Schließen des elektrischen Kontakts 25, wodurch ein elektrisches Signal erzeugt wird, welches die korrekte Positionierung (das Vorhandensein) eines Verbrauchsmittels 10 angibt. In der Ruheposition des Zapfens 22, d.h. ohne Verbrauchsmittel 10, ist der elektrische Kontakt 25 zwischen Kontaktfeder 27 und Federplatte 28 aufgrund des mechanischen Kontakts 19, der die Kontaktfeder 27 nach unten drückt, geöffnet, wodurch signalisiert wird, dass kein Verbrauchsmittel vorhanden ist. Bei Einschieben des Verbrauchsmittels 10 in den Förderweg wird also die Kontaktfeder 27 in Richtung der Federplatte 28 ausgelenkt und schließt den elektrischen Kontakt 25, so dass die Steuereinheit 40 ein elektrisches Schaltsignal erhält, das den Zustand korrekte Positionierung des Verbrauchselementes 10 repräsentiert. Der elektrische Kontakt 25, bestehend aus der Kontaktfeder 27 und der Federplatte 28, ist geschlossen.

Befindet sich kein analytisches Verbrauchsmittel 10 im Förderweg, ist der Zapfen 22 durch die Feder 24 in die Ruheposition verschoben, wobei er auf der Auflagefläche 29 aufliegt und somit die Kontaktfeder 27 nicht mehr gegen die Federplatte 28 drückt. Der elektrische Kontakt 25 ist geöffnet und kein elektrisches Schaltsignal wird an die Steuereinheit 40 übermittelt. Dies repräsentiert die Schaltinformation, dass sich kein analytisches Verbrauchsmittel 10 im Überwachungsbereich der elektrischen Schaltkomponente 21 befindet.

In diesem Schaltzustand ist der elektrische Kontakt 25 geöffnet. In diesem Zustand befindet sich das Analysehandgerät 1 regelmäßig dann, wenn es nicht benutzt wird und vom Benutzer transportiert wird. Gerade beim Transport in Taschen oder in der Kleidung besteht die Gefahr, dass Schmutzpartikel über Öffnungen des Gehäuses 4 des Analysehandgerätes 1 eindringen und im Inneren des Gehäuses 4 verbleiben. Besonders schädlich sind hierbei Schmutzpartikel, die im Bereich des elektrischen Kontakts 25 der elektrischen Schaltkomponente 21 zum Liegen kommen und dadurch die elektrische Kontaktierung der Kontaktfeder 27 mit der Federplatte 28 verhindern. In diesem Fall führt die Anwesenheit eines analytischen Verbrauchsmittels 10 zwar zu einer Auslenkung des Zapfens 22, was jedoch nicht zu einer Kontaktierung führen kann, da diese durch Schmutzpartikel verhindert ist. Mangels elektrischer Kontaktierung kann die Steuereinheit 40 keine Information über eine korrekte Positionierung bzw. das Vorhandensein des analytischen Verbrauchsmittels 10 erhalten. Auch die mechanische Bewegung des Zapfens 22 oder des mechanischen Kontakts 19 kann durch Schmutzpartikel behindert sein.

Die elektrische Schaltkomponente 21 ist als aktive optische Sensoreinheit 30 ausgebildet und umfasst eine LED 31, die Licht eines begrenzten Frequenzbereiches in Richtung des Verbrauchsmittels 10 emittiert. In einer nicht in Fig. 4 dargestellten Ausführungsform kann die optische Sensoreinheit 30 auf der Seite des Verbrauchsmittels 10 angeordnet sein, das derjenigen Seite gegenüberliegt, die der Auflagefläche 29 zugewandt ist. In Fig. 4 wäre die optische Sensoreinheit 30 dann oberhalb des Verbrauchsmittels 10, d.h. auf derselben Seite wie die elektrische Schaltkomponente 21, neben und unmittelbar benachbart zu dieser angeordnet. Der der optischen Sensoreinheit 30 zugewandte Bereich der Auflagefläche 29 wäre dann bevorzugt in dem Bereich der optischen Sensoreinheit 30 schwarz ausgebildet. Hierdurch wird nur wenig Licht von der Auflagefläche 29 zurück gestreut. Befindet sich in dem von der optischen Sensoreinheit 30 bestrahlten Bereich ein Verbrauchsmittel 10, das typischerweise eine helle, insbesondere weiße Oberfläche aufweist, beispielsweise ein im Rahmen der Analyse für Kalibrationszwecke genutztes weißes Feld (sogenannter Weißwertabgleich), so erhöht sich die Menge des reflektierten Lichts erheblich. Das reflektierte Licht wird durch einen Fotosensor 32 der optischen Sensoreinheit 30 erfasst. Diese beiden Zustände unterscheiden sich erheblich im Ausmaß des reflektierten und durch den Fotosensor 32 empfangenen Lichtes, so dass diese beiden Zustände sehr sicher mittels der optischen Sensoreinheit 30 unterschieden werden können. Diese gibt an die Steuereinheit 40 ein entsprechendes Sensorsignal ab, das entweder den Zustand korrekte Positionierung des Verbrauchsmittels 10 im Förderweg und damit auf der Auflagefläche 29 repräsentiert oder das den Zustand nicht vorhandenes Verbrauchsmittel 10 im Förderweg repräsentiert. Die Steuereinheit 40 wertet die beiden Signale der elektrischen Schaltkomponente 21 und der optischen Sensoreinheit 30 gemeinsam aus, wodurch eine Überprüfung bzw. Verifizierung des elektrischen Schaltsignals mit Hilfe des Sensorsignals erfolgt.

In der in Fig. 4 dargestellten Ausführungsform ist die optische Sensoreinheit 30 auf der Seite des Verbrauchsmittels 10 angeordnet, die der Auflagefläche 29 zugewandt ist. Die optische Sensoreinheit 30 ist also unterhalb des Verbrauchsmittels 10 angeordnet, d.h. auf der Seite des Verbrauchsmittels 10, die der elektrischen Schaltkomponente 21 gegenüberliegt. Die Auflagefläche 29 weist aus diesem Grund im Bereich der optischen Sensoreinheit 30 eine Öffnung oder einen durchsichtigen Fensterbereich 33 auf. Befindet sich in dem von der optischen Sensoreinheit 30 bestrahlten Bereich kein Verbrauchsmittel 10 wird nur wenig Streulicht zu dem Fotosensor 32 zurück gestreut. Befindet sich in dem von der optischen Sensoreinheit 30 bestrahlten Bereich jedoch ein Verbrauchsmittel 10, das typischerweise eine helle, insbesondere weiße Oberfläche aufweist, beispielsweise ein im Rahmen der Analyse für Kalibrationszwecke genutztes weißes Feld (sogenannter Weißwertabgleich), so erhöht sich die Menge des reflektierten Lichts erheblich. Das reflektierte Licht wird durch einen Fotosensor 32 der optischen Sensoreinheit 30 erfasst. Diese beiden Zustände unterscheiden sich erheblich im Ausmaß des reflektierten und durch den Fotosensor 32 empfangenen Lichtes, so dass diese beiden Zustände sehr sicher mittels der optischen Sensoreinheit 30 unterschieden werden können. Diese gibt an die Steuereinheit 40 ein entsprechendes Sensorsignal ab, das entweder den Zustand korrekte Positionierung des Verbrauchsmittels 10 im Förderweg und damit auf der Auflagefläche 29 repräsentiert oder das den Zustand nicht vorhandenes Verbrauchsmittel 10 im Förderweg repräsentiert. Die Steuereinheit 40 wertet die beiden Signale der elektrischen Schaltkomponente 21 und der optischen Sensoreinheit 30 gemeinsam aus, wodurch eine Überprüfung bzw. Verifizierung des elektrischen Schaltsignals mit Hilfe des Sensorsignals erfolgt.

Für beide im Zusammenhang mit Fig. 4 beschriebene Ausführungsformen wird nach dem Überprüfen der korrekten Positionierung eines Verbrauchsmittels 10 in der in Fig. 4 dargestellten Position des Verbrauchsmittels 10 der weitere Messablauf durchgeführt. Dabei kann in manchen Ausführungsformen unmittelbar in dieser Position des Verbrauchsmittels 10 die Analyse mit einem Analysesensor durchgeführt werden. Wenn jedoch die optische Sensoreinheit 30 gleichzeitig als Analysesensor dient oder der Analysesensor in die optische Sensoreinheit 30 integriert ist, ist es in der Regel erforderlich, das Verbrauchsmittel 10 aus der dargestellten Position in Pfeilrichtung in die Messposition weiterzufördern, bis sich beispielsweise ein fotometrisch vermessenes Testfeld im Sehbereich des Analysesensors befindet. In der Messposition des Verbrauchsmittels 10 kann dann das Ende 23 des Zapfens 22 in die Ausnehmung 26 eindringen. Dabei kann entweder der elektrische Kontakt 25 geschlossen bleiben und damit weiterhin die korrekte Positionierung, d.h. das Vorhandensein eines Verbrauchsmittels 10 signalisieren. In bevorzugten Ausführungsformen öffnet der elektrische Kontakt 25 jedoch in der Messposition des Verbrauchsmittels 10, um dadurch ein Signal zu erhalten, welches angibt, dass das Verbrauchsmittel 10 die Messposition erreicht hat. Die Tatsache, dass der geöffnete elektrische Kontakt 25 dann scheinbar angibt, dass kein Verbrauchsmittel 10 vorhanden ist, kann durch eine entsprechende Auflaufsteuerung mittels der Steuereinheit 40, in der zuvor die korrekte Positionierung des Verbrauchsmittels 10 in der Messposition verifiziert wurde, berücksichtigt werden.

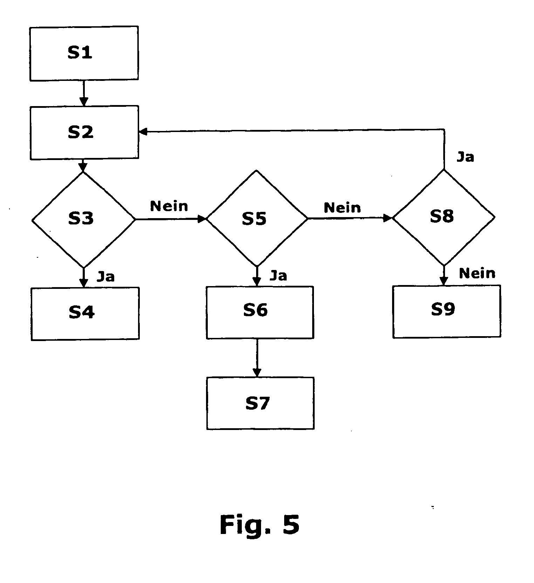

In Fig. 5 wird schematisch in Form eines Ablaufdiagramms beispielhaft die Überprüfung bzw. die Steuerung des Analysehandgerätes mit einem besonderen Augenmerk auf die Funktion der Prüfeinheit 20 dargestellt.

Nachdem in Schritt S1 durch Betätigung eines Tasters das Analysehandgerät 1 aktiviert und damit ein als Teststreifen ausgebildetes analytisches Verbrauchsmittel 10 angefordert wurde, wird im Schritt S2 mittels der Entnahmeeinrichtung 16 aus dem Trommelmagazin 6 ein streifenförmiges Verbrauchsmittel 10 entnommen und auf den Förderweg gebracht und auf diesem in Richtung des Analysesensors gefördert. Dabei erreicht das streifenförmige Verbrauchsmittel 10 den Bereich der elektrischen Schaltkomponente 21 bzw. der optischen Sensoreinheit 30. Durch das streifenförmige Verbrauchsmittel 10 wird der Zapfen 22 der elektrischen Schaltkomponente 21 verschoben und schließt dabei den elektrischen Kontakt zwischen der Federplatte 28 und der Kontaktfeder 27. Damit wird das Schaltsignal, welches den Zustand korrekte Positionierung des Verbrauchsmittels 10 repräsentiert, an die Steuereinheit 40 abgegeben. Diese Erfassung erfolgt im Schritt S3. Wird das Schaltsignal korrekte Positionierung des Verbrauchsmittels 10 durch die elektrische Schaltkomponente 21 an die Steuereinheit 40 abgegeben, so wird gemäß Schritt S4 der Analysesensor aktiviert und die Untersuchung der zu analysierenden Probe auf einen medizinisch bedeutsamen Bestandteil, beispielsweise Glukose, wird durchgeführt. Das Messergebnis wird anschließend mittels der Anzeigeeinrichtung 3 ausgegeben. Dieser Messablauf macht es sich zu Nutze, dass die Verlässlichkeit der Erfassung der elektrischen Schaltkomponente 21 für den Zustand korrekte Positionierung des Verbrauchsmittels 10 sehr hoch ist und daher auf einfache und direkte Art der Messvorgang durch den Analysesensor gestartet werden kann.

Wird gemäß Schritt S3 durch die elektrische Schaltkomponente 21 der Zustand keine korrekte Positionierung des Verbrauchsmittels 10 erfasst und an die Steuereinheit 40 weitergeleitet, so wird im Folgenden eine optische Erfassung der korrekten Positionierung mittels der optischen Sensoreinheit 30 vorgenommen. Dies erfolgt in Schritt S5. Die optische Sensoreinheit 30 ist ausschließlich in diesem Fall aktiviert, ansonsten ist sie deaktiviert. Die optische Sensoreinheit 30 wird also nur dann zum Überprüfen bzw. Verifizieren des Schaltsignals der elektrischen Schaltkomponente 21 aktiviert, wenn das Schaltsignal der elektrischen Schaltkomponente 21 angibt, es sei kein analytisches Verbrauchsmittel 10 in der korrekten Position auf dem Förderweg. Dadurch wird ein sehr energiesparender Betrieb des Analysehandgerätes 1 ermöglicht. Vorteilhafterweise wird die optische Sensoreinheit 30 automatisch zum Überprüfen bzw. Verifizieren des Schaltsignals der elektrischen Schaltkomponente 21 aktiviert, wenn die Entnahmeeinrichtung 16 zum Fördern eines analytischen Verbrauchsmittels 10 auf den Förderweg aktiviert worden ist, aber das Schaltsignal der elektrischen Schaltkomponente 21 angibt, es sei kein analytisches Verbrauchsmittel 10 in der korrekten Position auf dem Förderweg.

Wird nun im Schritt S5 festgestellt, dass kein Verbrauchsmittel 10 im Erfassungsbereich der optischen Sensoreinheit 30 vorliegt, so entspricht dies einer Bestätigung des elektrischen Schaltsignals, worauf die Steuereinheit 40 die Entnahmeeinrichtung 16 aktiviert, um aus der nächsten Kammer des Trommelmagazins 6 das nächste Verbrauchsmittel 10 zu entnehmen und in Richtung Analysesensor zu fördern. Dadurch ist sichergestellt, dass entsprechend der Anforderung gemäß Schritt S1 ein Verbrauchsmittel 10 gefördert wird, um die gewünschte Analyse vorzunehmen. Wird durch die Trommelprüfeinrichtung 18 oder eine Zähleinrichtung gemäß Schritt S8 festgestellt, dass die Trommel vollständig entleert ist und somit kein Verbrauchsmittel 10 mehr in dem Trommelmagazin 6 enthalten ist, so wird dies dem Benutzer mit Hilfe der Anzeigeeinrichtung 3 gemäß Schritt S9 mitgeteilt und der weitere Betrieb des Analysehandgerätes 1 eingestellt.

Wird durch die optische Sensoreinheit 30 eine korrekte Positionierung und damit das Vorhandensein des Verbrauchsmittels 10 gemäß Schritt S5 festgestellt und ein entsprechendes Sensorsignal an die Steuereinheit 40 abgegeben, so wird durch die Steuereinheit 40 der widersprüchliche Signalgehalt festgestellt und dies als Fehlfunktion des Analysehandgerätes 1 interpretiert. Diese Fehlfunktion zeichnet sich dadurch aus, dass ein streifenförmiges Verbrauchsmittel 10 im Bereich der elektrischen Schaltkomponente 21 und im Bereich des optischen Sensors 30 vorliegt, dies jedoch durch die elektrische Schaltkomponente 21 nicht erfasst wird und dadurch der Messvorgang gemäß Schritt S4 nicht gestartet wird. In diesem Fall wird gemäß Schritt S6 mittels der Anzeigeeinrichtung 3 eine Fehlermeldung ausgegeben, die auf diese Fehlfunktion hinweist und den Benutzer auffordert, eine Reinigung der elektrischen Kontakte 27, 28 der Schaltkomponente 21 vorzunehmen. Dies kann durch Starten eines entsprechenden Reinigungsprogramms des Analysehandgerätes 1 automatisiert erfolgen. Dadurch wird die Funktionsfähigkeit der elektrischen Schaltkomponente 21 wiederhergestellt und damit die Funktionsfähigkeit des Analysehandgerätes 1 wiedergewonnen.

Zusätzlich zu der Ausgabe der Fehlermeldung wird gemäß Schritt S7 das Verbrauchsmittel 10 aus dem Gerät ausgeschoben und dadurch die Möglichkeit geschaffen, nach erfolgter Reinigung zukünftig eine erfolgreiche Analyse vorzunehmen.

In dem Schritt S5 erfolgt somit insgesamt eine Auswertung des Schaltsignals der elektrischen Schaltkomponente 21 und des Sensorsignals der optischen Sensoreinheit 30, und in Abhängigkeit von dem Vergleich dieser Signale wird mittels der Steuereinheit 40 das Analysehandgerät 1 angesteuert. Durch den beschriebenen Betriebsablauf ist sichergestellt, dass die Fehlfunktionen des sogenannten Dauerstreifenschiebers, bei der fortlaufend Verbrauchsmittel 10 aus dem Trommelmagazin 6 mit Hilfe der Entnahmeeinrichtung 16 gefördert werden, ohne dass es zu einer Analyse kommt, bzw. die Fehlfunktion des Streifenklemmers, bei dem auf ein im Förderweg vorhandenes Verbrauchsmittel 10 ein weiteres Verbrauchsmittel 10 aufgeschoben wird, was zu einem Verkeilen der Verbrauchsmittel 10 im Förderweg führt, weitgehend verhindert wird.

Die vorstehend beschriebenen Verfahrensschritte können abgekürzt wie folgt charakterisiert werden: S1 Aktivierung Analysehandgerät, S2 Teststreifenentnahme, S3 Schaltsignalabfrage, S4 Aktivierung Analyse, S5 Sensorsignalabfrage, S6 Fehlermeldung, S7 Teststreifenausgabe, S8 Magazinvorratprüfung und S9 Magazin leer.

### Bezugszeichenliste

- 1: Analysehandgerät
- 2: Stromquelle
- 3: Anzeigeeinrichtung
- 4: Gehäuse
- 5: Ladeöffnung
- 6: Trommelmagazin
- 7: Magazinfach
- 9: Ausgabeöffnung
- 10: Verbrauchsmittel
- 12: Kammer
- 13: Entnahmeöffnung
- 14: Einschuböffnung
- 15: Stößel
- 16: Entnahmeeinrichtung
- 17: Siegelfolie
- 18: Trommelprüfeinrichtung
- 19: mechanischer Kontakt
- 20: Prüfeinheit
- 21: elektrische Schaltkomponente
- 22: Zapfen
- 23: konisch zulaufendes Ende
- 24: Feder
- 25: elektrischer Kontakt
- 26: Ausnehmung
- 27: Kontaktfeder
- 28: Federplatte
- 29: Auflagefläche
- 30: optische Sensoreinheit
- 31: LED
- 32: Fotosensor
- 33: Fensterbereich
- 40: Steuereinheit
- S1: Aktivierung Analysehandgerät
- S2: Teststreifenentnahme
- S3: Schaltsignalabfrage
- S4: Aktivierung Analyse
- S5: Sensorsignalabfrage
- S6: Fehlermeldung
- S7: Teststreifenausgabe
- S8: Magazinvorratprüfung
- S9: Magazin leer

## Patentansprüche

1. Analysehandgerät (1) zum Untersuchen einer Probe, insbesondere einer biologischen Flüssigkeit, auf einen medizinisch bedeutsamen Bestandteil, enthaltend
eine Anzeigeeinrichtung (3),
ein Gehäuse (4),
eine Ladeöffnung (5) zum Aufnehmen eines auswechselbaren Magazins (6), insbesondere eines Trommelmagazins, das analytische Verbrauchsmittel (10), insbesondere Teststreifen, enthalten kann,
eine Entnahmeeinrichtung (16) zum Entnehmen eines analytischen Verbrauchsmittels (10) aus dem Magazin (6) und zum Fördern auf einen Förderweg,
einen Analysesensor, dem auf dem Förderweg ein analytisches Verbrauchsmittel (10) zuführbar ist, und
eine Prüfeinheit (20), die eine korrekte Positionierung eines analytischen Verbrauchsmittels (10) im Förderweg feststellt,
**dadurch gekennzeichnet, dass**
die Prüfeinheit (20) sowohl
eine elektrische Schaltkomponente (21), die eine Positionierung eines analytischen Verbrauchsmittels (10) auf dem Förderweg mechanisch abtastet, die wenigstens eine Schaltposition einnimmt, die das Vorhandensein eines analytischen Verbrauchsmittels (10) repräsentiert, und die in Abhängigkeit von der Positionierung des analytischen Verbrauchsmittels (10) ein Schaltsignal abgibt,
als auch eine optische Sensoreinheit (30), die eine Positionierung eines analytischen Verbrauchsmittels (10) auf dem Förderweg optisch abtastet und die in Abhängigkeit von der Positionierung des analytischen Verbrauchsmittels (10) ein Sensorsignal abgibt,
und eine Steuereinheit (40) aufweist, die das Schaltsignal der elektrischen Schaltkomponente (21) und das Sensorsignal der optischen Sensoreinheit (30) auswertet und in Abhängigkeit von dem Vergleich dieser Signale das Analysehandgerät (1) ansteuert.

2. Analysehandgerät (1) nach Anspruch 1, **dadurch gekennzeichnet, dass** die elektrische Schaltkomponente (21) einen verschiebbar, insbesondere federnd gelagerten Zapfen (22) als Schaltelement mit einem dem analytischen Verbrauchsmittel (10) zugewandten konisch zulaufenden Ende (23) aufweist und das analytische Verbrauchsmittel (10) als positionsspezifische Oberflächengestaltung mindestens eine mit dem konisch zulaufenden Ende des Zapfens (22) korrespondierende Erhebung und/oder Ausnehmung (26) aufweist.

3. Analysehandgerät (1) nach Anspruch 2, **dadurch gekennzeichnet, dass** die positionsspezifische Oberflächengestaltung eine je nach Position des analytischen Verbrauchsmittels (10) im Förderweg die Auslenkung des als Schaltelement ausgebildeten Zapfens (22) beeinflussende Kontur, insbesondere eine in Breite und/oder Tiefe variierende Rinne oder eine in Breite und/oder Höhe variierende Rampe aufweist.

4. Analysehandgerät (1) nach einem der vorhergehenden Ansprüche, **dadurch gekennzeichnet, dass** die optische Sensoreinheit (30) zur Positionsbestimmung eines Schaltelements der elektrischen Schaltkomponente (21), insbesondere eines verschiebbaren Zapfens (22) ausgebildet ist.

5. Analysehandgerät (1) nach einem der Ansprüche 1 bis 3, **dadurch gekennzeichnet, dass** die optische Sensoreinheit (30) zum Bestimmen des Vorhandenseins und/oder der Position des analytischen Verbrauchsmittels (10) anhand dessen reflektierenden oder transmittierenden optischen Eigenschaften ausgebildet ist.

6. Analysehandgerät (1) nach einem der vorhergehenden Ansprüche, **dadurch gekennzeichnet, dass** die optische Sensoreinheit (30) mit dem Analysesensor eine gemeinsame Sensoreinheit bildet.

7. Analysehandgerät (1) nach einem der vorhergehenden Ansprüche, **dadurch gekennzeichnet, dass** das Entnehmen eines analytischen Verbrauchsmittels (10) aus dem Magazin (6) durch die Entnahmeeinrichtung (16) dann mittels der Steuereinheit (40) verhindert ist, wenn entweder das Schaltsignal der elektrischen Schaltkomponente (21) oder das Sensorsignal der optischen Sensoreinheit (30) eine korrekte Positionierung und das jeweils andere Signal eine nicht korrekte Positionierung eines analytischen Verbrauchsmittels (10) auf dem Förderweg repräsentiert, und dass vorzugsweise zusätzlich eine Fehlermeldung auf der Anzeigeeinrichtung (3) ausgegeben wird.

8. Analysehandgerät (1) nach einem der vorhergehenden Ansprüche, **dadurch gekennzeichnet, dass** der Analysesensor (15) zum Untersuchen einer Probe auf einen medizinisch bedeutsamen Bestandteil aktivierbar ist, wenn mittels eines Schaltsignals der elektrischen Schaltkomponente (21), das eine korrekte Positionierung eines analytischen Verbrauchsmittels (10) repräsentiert, eine korrekte Positionierung eines analytischen Verbrauchsmittels (10) im Förderweg festgestellt wurde.

9. Analysehandgerät (1) nach einem der vorhergehenden Ansprüche, **dadurch gekennzeichnet, dass** die optische Sensoreinheit (30) nur dann zum Überprüfen bzw. Verifizieren des Schaltsignals der elektrischen Schaltkomponente (21) aktivierbar ist, wenn das Schaltsignal der elektrischen Schaltkomponente (21) angibt, es sei kein analytisches Verbrauchsmittel (10) in der korrekten Position auf dem Förderweg.

10. Verfahren zum Betreiben eines Analysehandgeräts (1) zum Untersuchen einer Probe, insbesondere einer biologischen Flüssigkeit, auf einen medizinisch bedeutsamen Bestandteil, wobei das Analysehandgerät (1)
eine Anzeigeeinrichtung (3),
eine Entnahmeeinrichtung (16) zum Entnehmen eines analytischen Verbrauchsmittels (10) aus einem Magazin, insbesondere einem Trommelmagazin (6), und zum Fördern auf einen Förderweg,
einen Analysesensor, dem auf dem Förderweg ein analytisches Verbrauchsmittel (10) zuführbar ist, und
eine Prüfeinheit (20), die eine korrekte Positionierung eines analytischen Verbrauchsmittels (10) im Förderweg feststellt, umfasst,
**dadurch gekennzeichnet,**
**dass** nach einer Aktivierung des Analysehandgeräts (1)
und der darauf folgenden Entnahme eines analytischen Verbrauchsmittels (10) aus dem Magazin (6) und dem Fördern auf den Förderweg
Signale einer elektrischen Schaltkomponente (21), die eine Positionierung eines analytischen Verbrauchsmittels (10) auf dem Förderweg mechanisch abtastet, und einer optischen Sensoreinheit (30), die eine Positionierung eines analytischen Verbrauchsmittels (10) auf dem Förderweg optisch abtastet, mittels einer Steuereinheit (40) ausgewertet werden und in Abhängigkeit von dem Vergleich dieser Signale das Analysehandgerät (1) angesteuert wird.

11. Verfahren zum Betreiben eines Analysehandgeräts (1) nach Anspruch 10, **dadurch gekennzeichnet, dass** der Messvorgang durch den Analysesensor zum Untersuchen einer Probe auf einen medizinisch bedeutsamen Bestandteil freigegeben wird, wenn eine korrekte Positionierung durch die elektrische Schaltkomponente (21) signalisiert wurde.

12. Verfahren zum Betreiben eines Analysehandgeräts (1) nach einem der Ansprüche 10 bis 11, **dadurch gekennzeichnet, dass** die optische Sensoreinheit (30) nur dann zum Überprüfen bzw. Verifizieren des Schaltsignals der elektrischen Schaltkomponente (21) aktiviert wird, wenn das Schaltsignal der elektrischen Schaltkomponente (21) angibt, es sei kein analytisches Verbrauchsmittel (10) in der korrekten Position auf dem Förderweg.

13. Verfahren zum Betreiben eines Analysehandgeräts (1) nach einem der Ansprüche 10 bis 12, **dadurch gekennzeichnet, dass** die optische Sensoreinheit (30) automatisch zum Überprüfen bzw. Verifizieren des Schaltsignals der elektrischen Schaltkomponente (21) aktiviert wird, wenn die Entnahmeeinrichtung (16) zum Fördern eines analytischen Verbrauchsmittels (10) auf den Förderweg aktiviert worden ist, aber das Schaltsignal der elektrischen Schaltkomponente (21) angibt, es sei kein analytisches Verbrauchsmittel (10) in der korrekten Position auf dem Förderweg.

14. Verfahren zum Betreiben eines Analysehandgeräts (1) nach einem der Ansprüche 10 bis 13, **dadurch gekennzeichnet, dass** das Entnehmen eines analytischen Verbrauchsmittels (10) durch die Entnahmeeinrichtung (16) unterdrückt wird, wenn entweder das Signal der elektrischen Schaltkomponente (21) oder der optischen Sensoreinheit (30) eine korrekte Positionierung und das jeweils andere Signal eine nicht korrekte Positionierung eines analytischen Verbrauchsmittels (10) repräsentiert, und dass vorzugsweise zusätzlich eine Fehlermeldung auf der Anzeigeeinrichtung (3) ausgegeben wird.

15. Verfahren zum Betreiben eines Analysehandgeräts (1) nach einem der Ansprüche 10 bis 14, **dadurch gekennzeichnet, dass** die Entnahmeeinrichtung (16) aktiviert wird, wenn die elektrische Schaltkomponente (21) eine nicht korrekte Positionierung eines analytischen Verbrauchsmittels (10) festgestellt und die nicht korrekte Positionierung durch die optische Sensoreinheit (30) bestätigt wird.

16. Verfahren zum Betreiben eines Analysehandgeräts (1) nach Anspruch 15, **dadurch gekennzeichnet, dass** eine weitere Aktivierung der Entnahmeeinrichtung (16) verhindert wird und vorzugsweise eine Fehlermeldung ausgegeben wird, wenn eine vollständige Entleerung des Magazins (6) festgestellt wird.

## Claims

1. A handheld analyzer (1) for testing a sample, in particular a biological fluid, for a medically significant component, comprising
a display device (3),
a housing (4),
a loading opening (5) for receiving a replaceable magazine (6), in particular a rotary drum magazine that may contain analytical consumable means (10), in particular test strips,
a withdrawal device (16) for withdrawing an analytical consumable means (10) from the magazine (6) and for conveying it onto a conveyance pathway,
an analysis sensor to which an analytical consumable means (10) may be supplied on the conveyance pathway, and
a test unit (20) which detects the correct positioning of an analytical consumable means (10) in the conveyance pathway,
**characterized in that**
the test unit (20) comprises
an electric switch component (21), which mechanically senses the positioning of an analytical consumable means (10) on the conveyance pathway, assumes at least one switch position which represents the presence of an analytical consumable means (10) and delivers a switch signal as a function of the positioning of the analytical consumable means (10),
as well as an optical sensor unit (30), which optically senses the positioning of an analytical consumable means (10) on the conveyance pathway and delivers a sensor signal as a function of the positioning of the analytical consumable means (10),
and a control unit (40) which analyzes the switch signal of the electric switch component (21) and analyzes the sensor signal of the optical sensor unit (30) and controls the handheld analyzer (1) as a function of the comparison of these signals.

2. The handheld analyzer (1) according to claim 1, **characterized in that** the electric switch component (21) has a displaceable peg (22), in particular being spring-mounted, as a switch element with a conically tapering end (23) facing the analytical consumable means (10), and the analytical consumable means (10) has at least one elevation and/or recess (26) corresponding to the conically tapering end of the peg (22) as a position-specific surface design.

3. The handheld analyzer (1) according to claim 2, **characterized in that** the position-specific surface design has a contour that influences the deflection of the peg (22) which is designed as a switch element, depending on the position of the analytical consumable means (10) in the conveyance pathway, the contour being in particular a channel that varies in width and/or depth or a ramp that varies in width and/or height.

4. The handheld analyzer (1) according to any one of the preceding claims, **characterized in that** the optical sensor unit (30) is designed for determining the position of a switch element of the electric switch component (21), in particular of a displaceable peg (22).

5. The handheld analyzer (1) according to any one of claims 1 to 3, **characterized in that** the optical sensor unit (30) is designed for determining the presence and/or position of the analytical consumable means (10) on the basis of its reflective or transmitting optical properties.

6. The handheld analyzer (1) according to any one of the preceding claims, **characterized in that** the optical sensor unit (30) forms a joint sensor unit together with the analysis sensor.

7. The handheld analyzer (1) according to any one of the preceding claims, **characterized in that** the withdrawal of an analytical consumable means (10) from the magazine (6) by the withdrawal device (16) is prevented by the control unit (40) when either the switch signal of the electric switch component (21) or the sensor signal of the optical sensor unit (30) represents the correct positioning and the respective other signal represents the incorrect positioning of an analytical consumable means (10) on the conveyance pathway, and preferably an error message is additionally output on the display device (3).

8. The handheld analyzer (1) according to any one of the preceding claims, **characterized in that** the analysis sensor (15) can be activated to test a sample for a medically significant component when the correct positioning of an analytical consumable means (10) in the conveyance pathway has been detected by means of a switch signal of the electric switch component (21) which represents the correct positioning of an analytical consumable means (10).

9. The handheld analyzer (1) according to any one of the preceding claims, **characterized in that** the optical sensor unit (30) can be activated for checking and/or verifying the switch signal of the electric switch component (21) only when the switch signal of the electric switch component (21) indicates that there is no analytical consumable means (10) in the correct position on the conveyance pathway.

10. A method for operating a handheld analyzer (1) for testing a sample, in particular of a biological fluid, for a medically significant component, wherein the handheld analyzer (1) comprises
a display device (3),
a withdrawal device (16) for withdrawing an analytical consumable means (10) from a magazine, in particular a rotary drum magazine (6), and for conveying it onto a conveyance pathway,
an analysis sensor to which an analytical consumable means (10) may be supplied on the conveyance pathway, and
a test unit (20) which detects the correct positioning of an analytical consumable means (10) in the conveyance pathway,
**characterized in that**
after activation of the handheld analyzer (1)
and the subsequent withdrawal of an analytical consumable means (10) from the magazine (6) and its conveyance onto the conveyance pathway,
signals of an electric switch component (21) which mechanically senses the positioning of an analytical consumable means (10) on the conveyance pathway and of an optical sensor unit (30) which optically senses the positioning of the analytical consumable means (10) on the conveyance pathway are analyzed by means of a control unit (40), and the handheld analyzer (1) is controlled as a function of the comparison of these signals.

11. The method for operating a handheld analyzer (1) according to claim 10, **characterized in that** the measurement operation by the analysis sensor for testing a sample for a medically significant component is released when a correct positioning by the electric switch component (21) has been signaled.

12. The method for operating a handheld analyzer (1) according to any of claims 10 to 11, **characterized in that** the optical sensor unit (30) is activated for checking and/or verifying the switch signal of the electric switch component (21) only when the switch signal of the electric switch component (21) indicates that there is no analytical consumable means (10) in the correct position on the conveyance pathway.

13. The method for operating a handheld analyzer (1) according to any one of claims 10 to 12, **characterized in that** the optical sensor unit (30) is activated automatically for checking and/or verifying the switch signal of the electric switch component (21) when the withdrawal device (16) for conveying an analytical consumable means (10) onto the conveyance pathway has been activated but the switch signal of the electric switch component (21) indicates that there is no analytical consumable means (10) in the correct position on the conveyance pathway.

14. The method for operating a handheld analyzer (1) according to any one of claims 10 to 13, **characterized in that** the withdrawal of an analytical consumable means (10) by the withdrawal device (16) is suppressed when the signal of either the electric switch component (21) or of the optical sensor unit (30) represents the correct positioning of an analytical consumable means (10) and the other respective signal indicates incorrect positioning of an analytical consumable means (10), and that preferably an error message is additionally output on the display device (3).

15. The method for operating a handheld analyzer (1) according to any one of claims 10 to 14, **characterized in that** the withdrawal device (16) is activated when the electric switch component (21) detects that the positioning of the analytical consumable means (10) is not correct and the incorrect positioning is confirmed by the optical sensor unit (30).

16. The method for operating a handheld analyzer (1) according to claim 15, **characterized in that** further activation of the withdrawal device (16) is prevented and preferably an error message is output when complete emptying of the magazine (6) has been detected.

## Revendications

1. Appareil d'analyse portable (1) pour tester un échantillon, en particulier un liquide biologique, à l'égard d'un composant médicalement significatif, comprenant
un dispositif d'affichage (3),
un boîtier (4),
une ouverture de chargement (5) destinée à recevoir un magasin échangeable (6), en particulier un magasin à tambour pouvant contenir des consommables analytiques (10), notamment des bandelettes de test,
un dispositif de prélèvement (16) destiné à prélever un consommable analytique (10) dans le magasin (6) et à l'envoyer sur un parcours de transport,
un capteur d'analyse vers lequel un consommable analytique (10) peut être acheminé sur le parcours de transport, et
une unité de contrôle (20) qui constate un positionnement correct d'un consommable analytique (10) dans le parcours de transport,
**caractérisé en ce que**
l'unité de contrôle (20) comprend
un composant de commutation électrique (21), qui explore mécaniquement un positionnement d'un consommable analytique (10) sur le parcours de transport, qui prend au moins une position de commutation représentant la présence d'un consommable analytique (10) et qui délivre un signal de commutation en fonction du positionnement du consommable analytique (10),
ainsi qu'un module capteur optique (30), qui explore optiquement un positionnement d'un consommable analytique (10) sur le parcours de transport et qui délivre un signal capteur en fonction du positionnement du consommable analytique (10),
et une unité de commande (40), qui exploite le signal de commutation du composant de commutation électrique (21) et le signal capteur du module capteur optique (30) et commande l'appareil d'analyse portable (1) en fonction de la comparaison de ces signaux.

2. Appareil d'analyse portable (1) selon la revendication 1, **caractérisé en ce que** le composant de commutation électrique (21) comprend un doigt (22) monté coulissant, en particulier sur ressort, comme élément de commutation, avec une extrémité conique (23) tournée vers le consommable analytique (10), et le consommable analytique (10) présente comme conformation de surface spécifique à la position au moins une saillie et/ou un creux (26) correspondant à l'extrémité conique du doigt (22).

3. Appareil d'analyse portable (1) selon la revendication 2, **caractérisé en ce que** la conformation de surface spécifique à la position présente un contour qui influence la déflexion du doigt (22) conçu comme élément de commutation selon la position du consommable analytique (10) dans le parcours de transport, en particulier une rainure variable en largeur et/ou profondeur ou une rampe variable en largeur et/ou hauteur.

4. Appareil d'analyse portable (1) selon l'une des revendications précédentes, **caractérisé en ce que** le module capteur optique (30) est conçu pour déterminer la position d'un élément de commutation du composant de commutation électrique (21), en particulier d'un doigt coulissant (22).

5. Appareil d'analyse portable (1) selon l'une des revendications 1 à 3, **caractérisé en ce que** le module capteur optique (30) destiné à déterminer la présence et/ou la position du consommable analytique (10) est conçu à l'aide des propriétés optiques réfléchissantes ou transmettrices de ce dernier.

6. Appareil d'analyse portable (1) selon l'une des revendications précédentes, **caractérisé en ce que** le module capteur optique (30) forme un module capteur commun avec le capteur d'analyse.

7. Appareil d'analyse portable (1) selon l'une des revendications précédentes, **caractérisé en ce que** le prélèvement d'un consommable analytique (10) dans le magasin (6) par le dispositif de prélèvement (16) est empêché par l'unité de commande (40) lorsque soit le signal de commutation du composant de commutation électrique (21), soit le signal capteur du module capteur optique (30) représente un positionnement correct et l'autre signal respectif un positionnement non correct d'un consommable analytique (10), dans le parcours de transport et **en ce que** de préférence un message d'erreur s'affiche en plus sur le dispositif d'affichage (3).

8. Appareil d'analyse portable (1) selon l'une des revendications précédentes, **caractérisé en ce que** le capteur d'analyse (15) destiné à tester un échantillon à l'égard d'un composant médicalement significatif est activable lorsque, au moyen d'un signal de commutation du composant de commutation électrique (21) représentant un positionnement correct d'un consommable analytique (10), un positionnement correct d'un consommable analytique (10) dans le parcours de transport a été constaté.

9. Appareil d'analyse portable (1) selon l'une des revendications précédentes, **caractérisé en ce que** le module capteur optique (30) n'est activable pour contrôler, respectivement vérifier le signal de commutation du composant de commutation électrique (21) que lorsque le signal de commutation du composant de commutation électrique (21) indique qu'aucun consommable analytique (10) n'est en position correcte sur le parcours de transport.

10. Procédé permettant de faire fonctionner un appareil d'analyse portable (1) pour tester un échantillon, en particulier un liquide biologique, à l'égard d'un composant médicalement significatif, l'appareil d'analyse portable (1) comprenant un dispositif d'affichage (3),
un dispositif de prélèvement (16) destiné à prélever un consommable analytique (10) dans un magasin, en particulier un magasin à tambour (6), et à l'envoyer sur un parcours de transport,
un capteur d'analyse vers lequel un consommable analytique (10) peut être acheminé sur le parcours de transport, et
une unité de contrôle (20) qui constate un positionnement correct d'un consommable analytique (10) dans le parcours de transport,
**caractérisé en ce que**
après une activation de l'appareil d'analyse portable (1)
et le prélèvement subséquent d'un consommable analytique (10) dans le magasin (6) et son envoi sur le parcours de transport,
des signaux d'un composant de commutation électrique (21), lequel explore mécaniquement un positionnement d'un consommable analytique (10) sur le parcours de transport, et d'un module capteur optique (30), lequel explore optiquement un positionnement d'un consommable analytique (10) sur le parcours de transport, sont évalués au moyen d'une unité de commande (40) et l'appareil d'analyse portable (1) est commandé en fonction de la comparaison de ces signaux.

11. Procédé permettant de faire fonctionner un appareil d'analyse portable (1) selon la revendication 10, **caractérisé en ce que** le processus de mesure par le capteur d'analyse destiné à tester un échantillon à l'égard d'un composant médicalement significatif est autorisé lorsqu'un positionnement correct a été signalé par le composant de commutation électrique (21).

12. Procédé permettant de faire fonctionner un appareil d'analyse portable (1) selon l'une des revendications 10 à 11, **caractérisé en ce que** le module capteur optique (30) est activé automatiquement pour contrôler, respectivement vérifier le signal de commutation du composant de commutation électrique (21) lorsque le signal de commutation du composant de commutation électrique (21) indique qu'aucun consommable analytique (10) n'est en position correcte sur le parcours de transport.

13. Procédé permettant de faire fonctionner un appareil d'analyse portable (1) selon l'une des revendications 10 à 12, **caractérisé en ce que** le module capteur optique (30) est activé automatiquement pour contrôler, respectivement vérifier le signal de commutation du composant de commutation électrique (21) lorsque le dispositif de prélèvement (16) destiné à amener un consommable analytique (10) sur le parcours de transport a été activé mais que le signal de commutation du composant de commutation électrique (21) indique qu'aucun consommable analytique (10) n'est en position correcte sur le parcours de transport.

14. Procédé permettant de faire fonctionner un appareil d'analyse portable (1) selon l'une des revendications 10 à 13, **caractérisé en ce que** le prélèvement d'un consommable analytique (10) par le dispositif de prélèvement (16) est empêché lorsque soit le signal du composant de commutation électrique (21), soit celui du module capteur optique (30) représente un positionnement correct et l'autre signal respectif un positionnement non correct d'un consommable analytique (10), et **en ce que** de préférence un message d'erreur s'affiche en plus sur le dispositif d'affichage (3).

15. Procédé permettant de faire fonctionner un appareil d'analyse portable (1) selon l'une des revendications 10 à 14, **caractérisé en ce que** le dispositif de prélèvement (16) est activé lorsque le composant de commutation électrique (21) constate un positionnement non correct d'un consommable analytique (10) et que le positionnement non correct est confirmé par le module capteur optique (30).

16. Procédé permettant de faire fonctionner un appareil d'analyse portable (1) selon la revendication 15, **caractérisé en ce qu'**une nouvelle activation du dispositif de prélèvement (16) est empêchée et de préférence un message d'erreur est affiché lorsqu'il est constaté que le magasin (6) est totalement vide.
